# EUROPEAN PATENT SPECIFICATION

(11) **EP 3 005 331 B1**
(45) Date of publication and mention of the grant of the patent: **10.07.2019**
(21) Application number: 14725719.0
(22) Date of filing: 23.05.2014
(51) Int. Cl.: G08B 21/04, G08B 29/18, A61B 5/00, A61B 5/11

(54) **METHOD AND APPARATUS FOR DETERMINING THE RISK OF A PATIENT LEAVING A SAFE AREA**
VERFAHREN UND VORRICHTUNG ZUR BESTIMMUNG DES RISIKOS EINES PATIENTEN, EIN SICHERES GEBIET ZU VERLASSEN
PROCÉDÉ ET APPAREIL POUR DÉTERMINER LE RISQUE QU'UN PATIENT LAISSE UNE ZONE SÛRE

(30) Priority: 06.06.2013 EP 13170850
(43) Date of publication of application: 13.04.2016
(73) Proprietor: Koninklijke Philips N.V., 5656 AE Eindhoven (NL)
(72) Inventor: PIJL, Marten Jeroen, NL-5656 AE Eindhoven (NL); BALDUS, Heribert, NL-5656 AE Eindhoven (NL)
(74) Representative: Ledeboer, Johannes Albertus
(86) International application number: PCT/EP2014/060646
(87) International publication number: WO 2014/195149

(56) References cited:
- WO-A1-93/16636
- US-A1- 2003 140 060
- US-A1- 2006 071 798
- US-B1- 6 524 239
- US-B1- 8 441 356

## Description

### TECHNICAL FIELD OF THE INVENTION

The invention relates to a method and apparatus for monitoring a user, and in particular to a method and apparatus for monitoring a user that is located in a predefined safe area or zone.

### BACKGROUND TO THE INVENTION

It has been estimated that around 5.3 million Americans live with Alzheimer's disease, with the lifetime cost (direct and indirect) for care totaling over $100 billion annually. The number of people suffering from Alzheimer's disease or other forms of dementia is expected to increase considerably over the next few years. Incidents in which a person 'wanders' have been reported in 60% of people suffering from dementia, with 40% getting lost at least once during the course of the disease.

While there is no single accepted definition of wandering, it is described in "Evidence-based protocols for managing wandering behaviours" by A.L. Nelson and D. L. Algase as: "A syndrome of dementia-related locomotion behavior having a frequent, repetitive, temporally disordered, and/or spatially disoriented nature that is manifested in lapping, random, and/or pacing patterns some of which are associated with eloping, eloping attempts, or getting lost unless accompanied". Here, eloping refers to the person leaving a safe area, such as their home or an area defined around a care provider for the person. This wandering behavior can put the person at significant risk, and is a major source of stress for the care provider (for example a family member, neighbor or healthcare professional). Episodes of wandering behavior can often be a trigger for placing the person into a healthcare facility.

There are products available that can be used to monitor a person and detect eloping (wandering) in order to trigger an alarm and to track the movements of the person which allows care providers to locate the person when they are missing. A common technique is to use Global Positioning System (GPS) localization to detect the person's position relative to a predefined safe area. However, GPS is not effective until a person is outside (and potentially already wandering), due to the limited reception of the GPS satellite signals indoors. Alternatively, a number of sensors (such as cameras, RFID tag readers, etc.) can be installed in or around the safe area to detect entry into or exit from the safe area by the person. Again these techniques are potentially only effective once the person is attempting to leave (or has already left) the safe area, and often have other limitations such as complicated installation or operation, and leave open the possibility that the person could leave through an alternative route such as a window without being detected.

WO 93/16636 A1 discloses a monitoring device for a physical condition, including a wrist-held detector and transmitter unit and at least one separate receiver, to which a surveillance message or an alarm is delivered by means of a radio frequency message.

Thus, it is desirable to be able to detect a wandering event by a person before it becomes critical (i.e. before the person leaves the predefined safe area). Detecting a wandering event early means that eloping by the person may be avoided, or the person can be located earlier in case they leave the safe area. This will result in less stress and more reassurance for care providers, and less risk of injury for the person. A reliable way to detect wandering in an early stage can also help delay institutionalization for the person as a result of wandering, resulting in a better quality of life and reducing the societal and personal cost of dementia.

Thus, there is a need for a method and apparatus for monitoring a person that can provide an early warning (or earlier than conventional systems) that the person is likely to wander from a safe area.

### SUMMARY OF THE INVENTION

Very little is known about what causes specific episodes of wandering behavior. However, it has been found that wandering episodes are often accompanied by restlessness and/or agitation in the wanderer.

It is known in general that certain emotional states can produce a measurable physiological response in a person, such as a change in the conductivity of the skin through sweat production and/or an increase in the heart rate. These physiological characteristics, or changes in the measurements of physiological characteristics, can be measured using sensors that are worn or carried by a person.

The invention aims to measure one or more physiological characteristics of a user of a monitoring device, and use the measurements to estimate the user's emotional state (for example are they restless and/or agitated) and thus estimate the risk of the user having a wandering episode (i.e. leaving a predefined safe area for the user).

In a first aspect of the invention, there is provided a method of monitoring a user located in a predefined safe area, the method comprising measuring at least one physiological characteristic of the user; and processing the measurements of the at least one physiological characteristic to determine a risk of the user leaving the predefined safe area, the measurements of the at least one physiological characteristic being indicative of emotional state or affective state.

In some embodiments, the step of processing the measurements comprises comparing the measurements of the at least one physiological characteristic and/or one or more features extracted from the measurements of the at least one physiological characteristic to a threshold.

In alternative embodiments, the step of processing the measurements comprises comparing a change in the measurements of the at least one physiological characteristic and/or a change in one or more features extracted from the measurements of the at least one physiological characteristic to a threshold.

In yet further embodiments, the step of processing the measurements comprises processing the measurements using a machine learning algorithm.

In preferred embodiments, the method further comprises the steps of measuring the movements of the user; and processing the measured movements and the measurements of the at least one physiological characteristic to determine the risk of the user leaving the predefined safe area.

Preferably the step of measuring the movements of the user comprises measuring the movements using an accelerometer.

In some embodiments, the method further comprises the steps of determining the location of the user; and processing the determined location and the measurements of the at least one physiological characteristic to determine the risk of the user leaving the predefined safe area.

In some embodiments, the method further comprises the steps of storing information on the time or times that a user is expected to be at higher risk of leaving and/or has previously left the predefined safe area; and determining the risk of the user leaving the predefined safe area using the measurements of the at least one physiological characteristic, the current time and the stored information.

Preferably, the step of measuring at least one physiological characteristic of the user comprises measuring at least one of the skin conductivity and the heart rate of the user.

Preferably the method further comprises the step of triggering an alarm if the user is determined to be at high risk of leaving the predefined safe area.

In some embodiments, the method further comprises the step of determining the location of the user and transmitting the location to a care provider if the user is determined to be at high risk of leaving the predefined safe area.

The predefined safe area can be a geographical area or an area within a predefined distance of a care provider.

According to a second aspect of the invention, there is provided a computer program product comprising computer readable code embodied therein, the computer readable code being configured such that, on execution by a suitable computer or processing unit, the computer or processing unit performs any of the methods described above.

According to a third aspect of the invention, there is provided an apparatus for monitoring a user, the apparatus comprising a sensor for measuring at least one physiological characteristic of the user; and a processing unit for processing the measurements of the at least one physiological characteristic to determine a risk of the user leaving the predefined safe area, the measurements of the at least one physiological characteristic being indicative of emotional state or affective state.

In some embodiments, the processing unit is configured to process the measurements by comparing the measurements of the at least one physiological characteristic and/or one or more features extracted from the measurements of the at least one physiological characteristic to a threshold.

In alternative embodiments, the processing unit is configured to process the measurements by comparing a change in the measurements of the at least one physiological characteristic and/or a change in one or more features extracted from the measurements of the at least one physiological characteristic to a threshold.

In yet further embodiments, the processing unit is configured to process the measurements using a machine learning algorithm.

In preferred embodiments, the apparatus further comprises a sensor for measuring the movements of the user; and the processing unit is further configured to process the measured movements and the measurements of the at least one physiological characteristic to determine the risk of the user leaving the predefined safe area.

Preferably the sensor for measuring the movements of the user is an accelerometer.

In some embodiments, the apparatus further comprises means for determining the location of the user; and the processing unit is further configured to use the determined location and the measurements of the at least one physiological characteristic to determine the risk of the user leaving the predefined safe area.

In some embodiments, the apparatus further comprises a memory module for storing information on the time or times that a user is expected to be at higher risk of leaving and/or has previously left the predefined safe area; and the processing unit is further configured to determine the risk of the user leaving the predefined safe area using the measurements of the at least one physiological characteristic, the current time and the stored information.

Preferably, the sensor for measuring at least one physiological characteristic of the user is a sensor or sensors for measuring at least one of the skin conductivity and the heart rate of the user.

Preferably the processing unit is configured to trigger an alarm if the user is determined to be at high risk of leaving the predefined safe area.

In some embodiments, the apparatus further comprises means for determining the location of the user and transceiver circuitry for transmitting the determined location to a care provider if the processing unit determines that the user is at high risk of leaving the predefined safe area.

In some embodiments the sensor and processing unit are part of a monitoring device that is configured to be worn or carried by the user.

In alternative embodiments, the sensor is part of a monitoring device that is configured to be worn or carried by the user, and the processing unit is part of a base unit that is separate to the monitoring device.

### BRIEF DESCRIPTION OF THE DRAWINGS

Exemplary embodiments of the invention will now be described, by way of example only, with reference to the following drawings, in which:
Fig. 1 is a block diagram of a monitoring device according to an embodiment of the invention;
Fig. 2 is a flow chart illustrating a method of monitoring a user according to an embodiment of the invention; and
Fig. 3 is a flow chart illustrating a method of monitoring a user according to another embodiment of the invention.

### DETAILED DESCRIPTION OF THE PREFERRED EMBODIMENTS

As noted above, it is known that measurements of certain physiological characteristics can be used as estimates of the emotional state of a person, and where the measurements suggest that the person is in a restless, agitated or another emotional state associated with wandering events, it can be determined that the person is at a high or higher risk of having a wandering event (i.e. at high or higher risk of leaving a predefined safe area for the person). The predefined safe area can be a geographical area, such as within or around the person's home or their residential healthcare facility, or it can be defined as being within a certain distance of a base unit located in the person's home or residential healthcare facility or being within a certain distance of a care provider for the person.

The skin conductance (also known as the galvanic skin response, GSR) of a person is one such physiological characteristic that can be used to estimate the emotional state of the person. The skin conductance of a person can be measured at the wrist through a wrist-worn device. The GSR can be measured by observing the current passing between two electrodes in the wristband in contact with the skin. From this signal, changes in the person's affective state over time can be observed through changes in the skin conductance level, while more sudden changes are related to rises in the signal, called skin conductance rises (SCR).

Strong affective states such as agitation are associated with high arousal, which results in increased skin conductance levels or in SCRs. These values can be extracted from the GSR signal using signal analysis techniques, and if these values exceed some threshold or exceed the levels normally observed by some threshold, then in accordance with the invention this can indicate the person is in one of the affective states associated with wandering and is at high or higher risk of leaving the predefined safe area. Suitable signal analysis techniques for extracting these values from GSR signals are known in the art and will not be described in detail herein.

The heart rate of the person, or changes in the heart rate of the person can similarly be used as a measure of the person's emotional state, either in isolation or in combination with skin conductivity measurements.

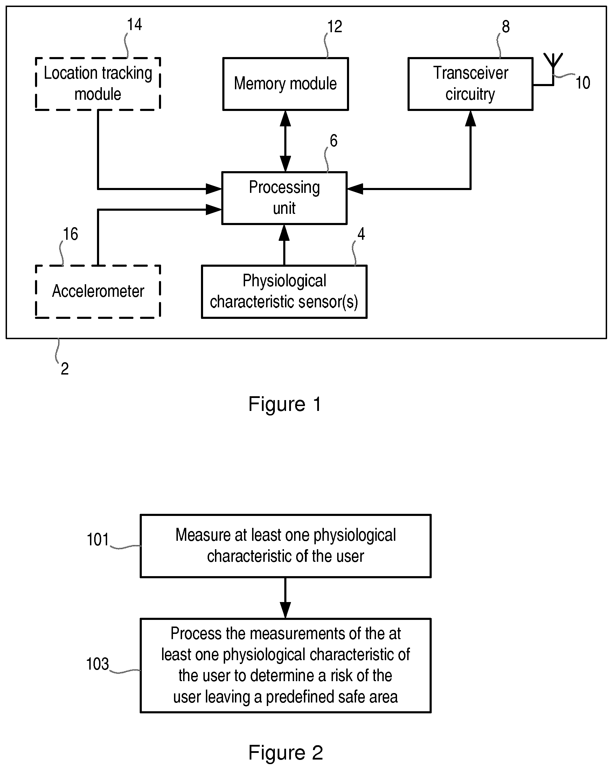

Thus, in accordance with the invention, an apparatus is provided in the form of a monitoring device that can measure one or more physiological characteristics of a user of the device. A monitoring device 2 according to an embodiment of the invention is shown in Fig. 1. The monitoring device 2 is designed to be worn or carried by a user (i.e. the person that may wander). In some embodiments the monitoring device 2 can be configured to be worn at the user's wrist or waist, on their chest or back, as a pendant on a cord or chain around their neck or carried in their pocket.

The monitoring device 2 comprises at least one sensor 4 for measuring a physiological characteristic of the user. Preferably the one or more sensors 4 measure at least one of skin conductivity (GSR) and heart rate of the user. Skin conductivity sensors are known in the art and will not be described in detail herein. Likewise, heart rate sensors are known in the art and known sensors include an electrocardiogram (ECG) sensor and a photoplethysmograph (PPG) sensor. In other embodiments, the signal from an accelerometer placed on the user's body will include accelerations caused by the beating of the heart and/or pumping of the blood around the user's body, and the acceleration signal can therefore be processed to extract the heart rate.

In some embodiments, the at least one sensor 4 can further comprise sensors for measuring the temperature of the user, their blood pressure, their breathing rate and/or any other physiological characteristic that can provide an indication of the emotional state of the user.

It will be appreciated that where the one or more sensors 4 require contact with the skin of the user in order to make the measurement (for example a skin conductivity sensor and certain types of heart rate sensor), the one or more sensors 4 will preferably be configured in the housing of the monitoring device 2 such that contact with the user's skin is formed when the device 2 is being worn by the user. In alternative embodiments, particularly where the form factor of the monitoring device 4 does not provide consistent contact with the user's skin (such as when the monitoring device 4 is in the form of a pendant), the part of the sensor that needs to be in contact with the user's skin (e.g. an electrode) can be separately attached to the user's skin using, for example, an adhesive patch or a band or strap.

The monitoring device 2 also comprises a processing unit 6 which receives measurements from the one or more sensors 4 and processes the measurements to determine the risk of the user leaving the predefined safe area. The processing unit 6 also generally controls the operation of the monitoring device 2.

The monitoring device 2 also comprises transceiver circuitry 8 that is connected to processing unit 6 and an associated antenna 10 that allows the monitoring device 2 to communicate with other electronic devices. For example, if it is determined that the user is at a high or higher risk of leaving the predefined safe area, the processing unit 6 can transmit an alarm signal via the transceiver circuitry 8 and antenna 10 to a remote call centre or a remote device (such as a pager, smartphone, tablet, computer, etc.) belonging to a specified care provider to alert them that the user may be about to wander out of the predefined safe area. The transceiver circuitry 8 can use any suitable type of wireless communications protocol to communicate with the other devices, such as, for example, GSM, WCDMA, UMTS, LTE, Bluetooth, Wi-Fi, etc.

The monitoring device 2 also comprises a memory module 12 that is connected to the processing unit 6 and that can store measurement data from the one or more sensors 4 prior to processing by the processing unit 6, and/or computer readable code for use by the processing unit 6.

In some embodiments, the monitoring device 4 can comprise a location tracking module 14 connected to the processing unit 6 that is for determining the position or location of the monitoring device 4. For example the module 14 can comprise a satellite positioning system (SPS) receiver, such as a GPS, GNSS, GLONASS or Galileo receiver. However, those skilled in the art will be aware of other techniques for determining the location of the user, including, for example, techniques that use cell-tower triangulation and/or the identities of nearby Wi-Fi access points, and the module 14 can comprise suitable components for determining the location of the user using these techniques. In some embodiments the module 14 can be selectively activated if the user is determined to be at (high) risk of leaving the predefined safe area in order to start tracking the user's location, and in other embodiments the module 14 can be activated intermittently to determine the user's location (for example to determine the location with reference to the predefined safe area to detect wandering events). The position or location of the monitoring device 4 output by the module 14 can be transmitted to the remote call centre or remote care provider device using the transceiver circuitry 8.

The monitoring device 2 can also optionally comprise an accelerometer 16 that measures (preferably in three dimensions) the accelerations experienced by the monitoring device 2. The processing unit 6 can analyze the accelerations to estimate the activity level of the user.

In alternative embodiments to that shown in Fig. 1, the monitoring device 2 can be part of a system that comprises a base unit that can be located in the home (or residential healthcare facility) of the user and that communicates wirelessly with the monitoring device 2. The base unit may also act as a charging station for the monitoring device 2 when it is not in use. The base unit may comprise circuitry for enabling communications between the monitoring device 2 and a remote call centre or a remote care provider device via a public switched telephone network and/or a mobile communications network, and/or may provide a connection to the Internet. In some implementations of this system, the processing and operations according to the invention can be performed by the processing unit 6 in the monitoring device 2, with the base unit being provided merely to facilitate communications with the remote call centre/care provider device. In alternative implementations, the monitoring device 2 can communicate the measurements obtained by the one or more physiological characteristic sensors 4 to the base unit, and a processing unit in the base unit can perform the processing and operations according to the invention using the measurements. This latter embodiment has the advantage that the power consumption of the monitoring device 2 can be substantially reduced.

In the embodiments where the monitoring device 2 communicates with a base unit, the communication can be made using any known wireless technology, for example Wi-Fi, Bluetooth, Near Field Communication (NFC), etc.

The flow chart in Fig. 2 illustrates a method of monitoring the user according to an embodiment of the invention. In step 101, at least one physiological characteristic of the user is measured using the monitoring device 2 described above. The at least one physiological characteristic may comprise the conductivity of the user's skin as measured at the wrist or other part of the user's body, and/or the user's heart rate. In some embodiments, step 101 can comprise collecting an instantaneous measurement of the physiological characteristic, while in other embodiments step 101 can comprise collecting a time series of measurements of the physiological characteristic.

Then, in step 103, the measurements of the at least physiological characteristic are processed to determine the risk of the user leaving a predefined safe area. As noted above, this processing can be performed by processing unit 6 in the monitoring device 2 or it can be performed by a processing unit in a separate base unit.

In some embodiments, the processing in step 103 comprises simply comparing the measurement of the physiological characteristic, or a feature extracted from the measurements (such as the occurrence of skin conductance rises, SCRs) to a threshold, and determining that the user is at high risk of leaving the predefined safe area if the measurement exceeds or falls below the threshold (as appropriate for the particular physiological characteristic). Thus, in one embodiment, the user can be determined to be a high risk of leaving the predefined safe area if the measured skin conductivity is above a threshold (since higher skin conductivity suggests the user is more stressed/agitated).

In other embodiments, the processing in step 103 can comprise determining a change in the physiological characteristic from a normal or average value for the user or a change in the physiological characteristic over a certain time period (e.g. 5 minutes), and comparing the change to a threshold.

If the user is determined to be at high risk of leaving the predefined safe area, then the method can further comprise triggering an alarm in which an alert is sent to a remote call centre and/or to an electronic device belonging to a care provider for the user. Alternatively or in addition, a location tracking module 14 in the monitoring device 2 can be activated and the tracked-location sent to the remote call centre and/or care provider in order to allow the user to be found if they leave the predefined safe area.

In further embodiments described in more detail below, the measurements of the physiological characteristic(s) can be processed in combination with measurements from other sensors and/or other information in order to determine the risk of the user leaving the predefined safe area.

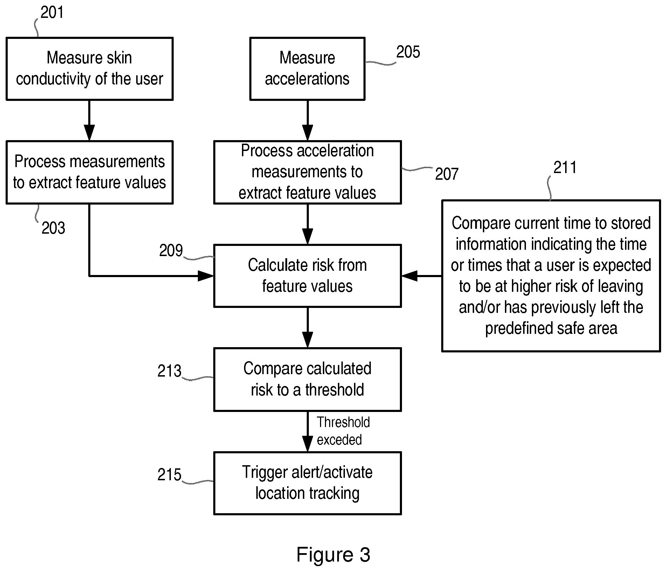

The flow chart in Fig. 3 illustrates a method of monitoring a user according to another embodiment of the invention. In a first step, step 201, the skin conductivity of the user is measured, and the measurements of the skin conductivity are processed to extract one or more feature values (step 203) that are to be used in the risk calculation. As noted above, the feature values can include the occurrence of skin conductance rises (SCR) in the measurements or the highest measured skin conductivity during a preceding time period. Other feature values that can be extracted include the mean or standard deviation of the skin conductance level, the amplitude, gradient, rise duration, recovery half-time or count of the SCRs.

At the same time or around the same time that the skin conductivity measurements are taken, the accelerations of the monitoring device 2 (and thus the accelerations of the user) are measured using accelerometer 16 (step 205). In step 207 the acceleration measurements are processed to extract feature values related to the physical activity and activity levels of the user. Other feature values that can be extracted include the magnitude of the acceleration, vertical and/or lateral movement, orientation (based on the measured direction of gravity), the number of peaks and frequency-based measures.

The feature values extracted from the skin conductivity measurements and the acceleration measurements (and in further embodiments feature values extracted from other physiological characteristic measurements, such as heart rate, temperature, etc.) are processed to calculate the risk of the user wandering from the predefined safe area (step 209).

Processing skin conductivity measurements in conjunction with acceleration measurements (and in particular values for the user's activity level that are extracted from the acceleration measurements) to determine a risk of the user leaving the predefined safe area is advantageous. In a first case, observing skin conductivity alone can lead to false alarms since intense physical activity such as exercising increases skin conductance levels, and this would not typically lead to a wandering event. The amount of activity can be monitored using the signal from the accelerometer 16 and a number of false alarms can be prevented. In particular, a threshold or criteria applied to the skin conductivity measurements to determine if the skin conductance is abnormal (and thus an indicator of imminent wandering behavior) can be adapted based on the amount of motion observed in the signal from the accelerometer 16. For example if the amount of motion or activity is above a threshold, the criteria applied to the skin conductivity measurements might require a sharper gradient in SCRs, or higher absolute skin conductivity levels. This would allow wandering events to be detected in high activity situations, but reduce false alarms.

In a second case, the confidence in detecting a wandering event can be increased by observing both a significant change in skin conductivity (due to increased activity of the user) and processing the signal from the accelerometer 16 to detect if the user is walking or at rest (for example through the use of step detection algorithms). Since wandering includes a locomotion aspect, an indication from the skin conductivity measurements that the user may be at high risk of wandering can be disregarded if the activity level suggests that the user is at rest. In another case, if the activity level suggests that the user is walking, the threshold applied to the feature value extracted from the skin conductivity measurements can be adjusted to make detection of wandering more likely.

The processing in step 209 can comprise applying a number of thresholds to the extracted features. For example respective thresholds can be applied to the feature value(s) extracted from the skin conductivity measurements and the feature value(s) extracted from the acceleration measurements, and if the values are above the respective thresholds then the user can be determined to be at high risk of wandering.

Alternatively, multiple thresholds can be applied to the feature values to provide a graded risk value (e.g. low, medium, high), or a risk value can be determined based on the amount by which the feature values exceed the thresholds.

As another alternative, each of the feature values can be individually used to derive a risk value, and risk values for all of the feature values accumulated to give an overall risk value.

As yet another alternative, the feature values can be processed using machine learning algorithms, such as a Bayesian belief network, that can adapt the feature values and/or risk values based on observed events or differences between different users (for example differences in baseline skin conductivity or heart rate for the user). In these embodiments, the machine learning algorithm can also record patterns of feature values from the measurements of the skin conductivity and activity level (and other features), and use these to train the algorithm to reduce the occurrence of false alarms and false negatives. In particular the algorithm can match certain feature values or sets of feature values with the probability of a wandering event occurring. In these embodiments indications should be provided to the algorithm when wandering events occur (which could be provided, for example, by the care provider using their electronic device) to enable the algorithm to identify the appropriate feature value patterns.

A further feature that can be used to derive the risk value for the user is based on the time of day that the user is most likely to wander. Thus, in step 211, the current time is compared to information indicating the time or times of day that the user is most likely to wander. This information may be derived from times that the user has previously wandered out of the predefined safe area (with the times being provided by, for example, the care provider). In addition or alternatively, this information can be based on times that users are typically at higher risk of wandering. For example, it has been found that in general people are more likely to wander at dusk than at other times of the day. The result of the comparison in step 211 is input to step 209 and the risk value is calculated using the result of the comparison and the other feature values.

The risk value determined in step 209 (even if represented as a simple 'high' or 'low' risk) is then compared to a risk threshold (step 213), and if the risk value exceeds the risk threshold then the user is deemed to be at risk of leaving the predefined area and an alert is triggered (step 215). As noted above, triggering an alert can involve sending a message to an electronic device carried by a care provider and/or sending a message to a remote call centre. Also as noted above, a location tracking module 14 in the monitoring device 2 can be activated and the location of the user transmitted to the care provider device and/or remote call centre in order to allow the user to be found quickly.

There is therefore provided a method and apparatus for monitoring a user that can provide an early warning (or earlier than conventional systems) that the user is likely to wander from a safe area.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of monitoring a user located in a predefined safe area, the method comprising:
measuring (101) at least one physiological characteristic of the user; and
**characterized in that** the method further comprising processing (103) the measurements of the at least one physiological characteristic to determine a risk of the user leaving the predefined safe area, the measurements of the at least one physiological characteristic being indicative of emotional state or affective state.

2. A method as claimed in claim 1, wherein the step of processing the measurements comprises comparing the measurements of the at least one physiological characteristic and/or one or more features extracted from the measurements of the at least one physiological characteristic to a threshold.

3. A method as claimed in claim 1, wherein the step of processing the measurements comprises comparing a change in the measurements of the at least one physiological characteristic and/or a change in one or more features extracted from the measurements of the at least one physiological characteristic to a threshold.

4. A method as claimed in claim 1, wherein the step of processing the measurements comprises processing the measurements using a machine learning algorithm.

5. A method as claimed in any preceding claim, further comprising the steps of:
measuring the movements of the user; and
processing the measured movements and the measurements of the at least one physiological characteristic to determine the risk of the user leaving the predefined safe area.

6. A method as claimed in any preceding claim, further comprising the steps of:
determining the location of the user; and
processing the determined location and the measurements of the at least one physiological characteristic to determine the risk of the user leaving the predefined safe area.

7. A method as claimed in any preceding claim, further comprising the steps of:
storing information on the time or times that a user is expected to be at higher risk of leaving and/or has previously left the predefined safe area; and
determining the risk of the user leaving the predefined safe area using the measurements of the at least one physiological characteristic, the current time and the stored information.

8. A method as claimed in any preceding claim, wherein the step of measuring at least one physiological characteristic of the user comprises measuring at least one of the skin conductivity and the heart rate of the user.

9. A computer program product comprising computer readable code embodied therein, the computer readable code being configured such that, on execution by a suitable computer or processing unit, the computer or processing unit performs the method of any of claims 1 to 8.

10. An apparatus (2) for monitoring a user, the apparatus comprising:
a sensor (4) for measuring at least one physiological characteristic of the user; and **characterized in that** the apparatus further comprising a processing unit (6) for processing the measurements of the at least one physiological characteristic to determine a risk of the user leaving the predefined safe area, the measurements of the at least one physiological characteristic being indicative of emotional state or affective state.

11. An apparatus (2) as claimed in claim 10, the apparatus further comprising:
a sensor (16) for measuring the movements of the user;
wherein the processing unit (6) is further configured to process the measured movements and the measurements of the at least one physiological characteristic to determine the risk of the user leaving the predefined safe area.

12. An apparatus (2) as claimed in claim 10 or 11, the apparatus further comprising:
means (14) for determining the location of the user;
wherein the processing unit (6) is further configured to use the determined location and the measurements of the at least one physiological characteristic to determine the risk of the user leaving the predefined safe area.

13. An apparatus (2) as claimed in claim 10, 11 or 12, the apparatus further comprising:
a memory module (12) for storing information on the time or times that a user is expected to be at higher risk of leaving and/or has previously left the predefined safe area; wherein the processing unit (6) is further configured to determine the risk of the user leaving the predefined safe area using the measurements of the at least one physiological characteristic, the current time and the stored information.

14. An apparatus (2) as claimed in any of claims 10 to 13, wherein the sensor (4) for measuring at least one physiological characteristic of the user is a sensor or sensors for measuring at least one of the skin conductivity and the heart rate of the user.

15. An apparatus (2) as claimed in any of claims 10 to 14, the apparatus further comprising:
means (14) for determining the location of the user and transceiver circuitry (8) for transmitting the determined location to a care provider if the processing unit (6) determines that the user is at high risk of leaving the predefined safe area.

## Patentansprüche

1. Verfahren zum Überwachen eines Nutzers, der sich in einem vorab definierten sicheren Bereich befindet, wobei das Verfahren Folgendes umfasst:
Messen (101) mindestens eines physiologischen Merkmals des Nutzers; und
**dadurch gekennzeichnet, dass** das Verfahren weiter das Verarbeiten (103) der Messungen des mindestens einen physiologischen Merkmals umfasst, um ein Risiko, dass der Nutzer den vorab definierten sicheren Bereich verlässt, zu ermitteln, wobei die Messungen des mindestens einen physiologischen Merkmals für einen emotionalen Zustand oder einen affektiven Zustand indikativ sind.

2. Verfahren nach Anspruch 1, wobei der Schritt des Verarbeitens der Messungen das Vergleichen der Messungen des mindestens einen physiologischen Merkmals und/oder eines oder mehrerer aus den Messungen des mindestens einen physiologischen Merkmals extrahierter Merkmale mit einem Grenzwert umfasst.

3. Verfahren nach Anspruch 1, wobei der Schritt des Verarbeitens der Messungen das Vergleichen einer Veränderung bei den Messungen des mindestens einen physiologischen Merkmals und/oder einer Veränderung bei einem oder mehreren aus den Messungen des mindestens einen physiologischen Merkmals extrahierten Merkmalen mit einem Grenzwert umfasst.

4. Verfahren nach Anspruch 1, wobei der Schritt des Verarbeitens der Messungen das Verarbeiten der Messungen unter Verwendung eines Algorithmus für maschinelles Lernen umfasst.

5. Verfahren nach einem der vorstehenden Ansprüche, weiter umfassend die folgenden Schritte:
Messen der Bewegungen des Nutzers; und
Verarbeiten der gemessenen Bewegungen und der Messungen des mindestens einen physiologischen Merkmals, um das Risiko, dass der Nutzer den vorab definierten sicheren Bereich verlässt, zu ermitteln.

6. Verfahren nach einem der vorstehenden Ansprüche, weiter umfassend die folgenden Schritte:
Ermitteln des Standorts des Nutzers; und
Verarbeiten des ermittelten Standorts und der Messungen des mindestens einen physiologischen Merkmals, um das Risiko, dass der Nutzer den vorab definierten sicheren Bereich verlässt, zu ermitteln.

7. Verfahren nach einem der vorstehenden Ansprüche, weiter umfassend die folgenden Schritte:
Speichern von Informationen über den Zeitpunkt oder die Zeitpunkte, zu dem/denen das Risiko, dass ein Nutzer den vorab definierten sicheren Bereich verlässt, voraussichtlich höher ist und/oder ein Nutzer den vorab definierten sicheren Bereich vorher verlassen hat; und
Ermitteln des Risikos, dass der Nutzer den vorab definierten sicheren Bereich verlässt, unter Verwendung der Messungen des mindestens einen physiologischen Merkmals, des aktuellen Zeitpunkts und der gespeicherten Informationen.

8. Verfahren nach einem der vorstehenden Ansprüche, wobei der Schritt des Messens mindestens eines physiologischen Merkmals des Nutzers das Messen von mindestens einer der Hautleitfähigkeit und der Herzfrequenz des Nutzers umfasst.

9. Computerprogrammprodukt, umfassend einen darin enthaltenen computerlesbaren Code, wobei der computerlesbare Code so konfiguriert ist, dass bei Ausführung durch einen geeigneten Computer oder eine geeignete Verarbeitungseinheit der Computer oder die Verarbeitungseinheit das Verfahren nach einem der Ansprüche 1 bis 8 durchführt.

10. Vorrichtung (2) zum Überwachen eines Nutzers, wobei die Vorrichtung Folgendes umfasst:
einen Sensor (4) zum Messen mindestens eines physiologischen Merkmals des Nutzers;
und **dadurch gekennzeichnet, dass** die Vorrichtung weiter eine Verarbeitungseinheit (6) zum Verarbeiten der Messungen des mindestens einen physiologischen Merkmals umfasst, um ein Risiko, dass der Nutzer den vorab definierten sicheren Bereich verlässt, zu ermitteln, wobei die Messungen des mindestens einen physiologischen Merkmals für einen emotionalen Zustand oder einen affektiven Zustand indikativ sind.

11. Vorrichtung (2) nach Anspruch 10, wobei die Vorrichtung weiter Folgendes umfasst:
einen Sensor (16) zum Messen der Bewegungen des Nutzers;
wobei die Verarbeitungseinheit (6) weiter konfiguriert ist, die gemessenen Bewegungen und die Messungen des mindestens einen physiologischen Merkmals zu verarbeiten, um das Risiko, dass der Nutzer den vorab definierten sicheren Bereich verlässt, zu ermitteln.

12. Vorrichtung (2) nach Anspruch 10 oder 11, wobei die Vorrichtung weiter Folgendes umfasst:
ein Mittel (14) zum Ermitteln des Standorts des Nutzers;
wobei die Verarbeitungseinheit (6) weiter konfiguriert ist, den ermittelten Standort und die Messungen des mindestens einen physiologischen Merkmals zu verwenden, um das Risiko, dass der Nutzer den vorab definierten sicheren Bereich verlässt, zu ermitteln.

13. Vorrichtung (2) nach Anspruch 10, 11 oder 12, wobei die Vorrichtung weiter Folgendes umfasst:
ein Speichermodul (12) zum Speichern von Informationen über den Zeitpunkt oder die Zeitpunkte, zu dem/denen das Risiko, dass ein Nutzer den vorab definierten sicheren Bereich verlässt, voraussichtlich höher ist und/oder ein Nutzer den vorab definierten sicheren Bereich vorher verlassen hat;
wobei die Verarbeitungseinheit (6) weiter konfiguriert ist, das Risiko, dass der Nutzer den vorab definierten sicheren Bereich verlässt, unter Verwendung der Messungen des mindestens einen physiologischen Merkmals, des aktuellen Zeitpunkts und der gespeicherten Informationen zu ermitteln.

14. Vorrichtung (2) nach einem der Ansprüche 10 bis 13, wobei der Sensor (4) zum Messen mindestens eines physiologischen Merkmals des Nutzers ein Sensor oder Sensoren zum Messen von mindestens einer der Hautleitfähigkeit und der Herzfrequenz des Nutzers ist.

15. Vorrichtung (2) nach einem der Ansprüche 10 bis 14, wobei die Vorrichtung weiter Folgendes umfasst:
ein Mittel (14) zum Ermitteln des Standorts des Nutzers und eine Sender-Empfängerschaltung (8) zum Übertragen des ermittelten Standorts an einen Pflegedienstleister, wenn die Verarbeitungseinheit (6) ermittelt, dass das Risiko, dass der Nutzer den vorab definierten sicheren Bereich verlässt, hoch ist.

## Revendications

1. Procédé de surveillance d'un utilisateur situé dans une zone sûre prédéfinie, le procédé comprenant :
la mesure (101) d'au moins une caractéristique physiologique de l'utilisateur ; et
**caractérisé en ce que** le procédé comprend en outre le traitement (103) des mesures de l'au moins une caractéristique physiologique pour déterminer un risque que l'utilisateur quitte la zone sûre prédéfinie, les mesures de l'au moins une caractéristique physiologique représentant un état émotionnel ou un état affectif.

2. Procédé selon la revendication 1, dans lequel l'étape de traitement des mesures comprend la comparaison des mesures de l'au moins une caractéristique physiologique et/ou d'un ou plusieurs éléments extraits des mesures de l'au moins une caractéristique physiologique par rapport à un seuil.

3. Procédé selon la revendication 1, dans lequel l'étape de traitement des mesures comprend la comparaison d'une variation des mesures de l'au moins une caractéristique physiologique et/ou d'une variation d'un ou plusieurs éléments extraits des mesures de l'au moins une caractéristique physiologique par rapport à un seuil.

4. Procédé selon la revendication 1, dans lequel l'étape de traitement des mesures comprend le traitement des mesures en utilisant un algorithme d'apprentissage automatique.

5. Procédé selon l'une quelconque des revendications précédentes, comprenant en outre les étapes de :
mesure des déplacements de l'utilisateur ; et
traitement des déplacements mesurés et des mesures de l'au moins une caractéristique physiologique pour déterminer le risque que l'utilisateur quitte la zone sûre prédéfinie.

6. Procédé selon l'une quelconque des revendications précédentes, comprenant en outre les étapes de :
détermination de l'emplacement de l'utilisateur ; et
traitement de l'emplacement déterminé et des mesures de l'au moins une caractéristique physiologique pour déterminer le risque que l'utilisateur quitte la zone sûre prédéfinie.

7. Procédé selon l'une quelconque des revendications précédentes, comprenant en outre les étapes de :
stockage d'informations relatives à l'heure ou aux heures où l'utilisateur est supposé présenter un risque plus élevé de quitter et/ou a déjà quitté la zone sûre prédéfinie ;
détermination du risque que l'utilisateur quitte la zone sûre prédéfinie en utilisant les mesures de l'au moins une caractéristique physiologique, l'heure et les informations stockées.

8. Procédé selon l'une quelconque des revendications précédentes, dans lequel l'étape de mesure d'au moins une caractéristique physiologique de l'utilisateur comprend la mesure d'au moins l'un parmi la conductance cutanée et le rythme cardiaque de l'utilisateur.

9. Produit de programme informatique comprenant un code lisible par ordinateur intégré dans celui-ci, le code lisible par ordinateur étant configuré de sorte que, lors de l'exécution par un ordinateur ou unité de traitement approprié, l'ordinateur ou l'unité de traitement mette en oeuvre le procédé selon l'une quelconque des revendications 1 à 8.

10. Appareil (2) pour surveiller un utilisateur, l'appareil comprenant :
un capteur (4) pour mesurer au moins une caractéristique physiologique de l'utilisateur ; et **caractérisé en ce que** l'appareil comprend en outre une unité de traitement (6) pour traiter les mesures de l'au moins une caractéristique physiologique pour déterminer un risque que l'utilisateur quitte la zone sûre prédéfinie, les mesures de l'au moins une caractéristique physiologique représentant un état émotionnel ou un état affectif.

11. Appareil (2) selon la revendication 10, l'appareil comprenant en outre :
un capteur (16) pour mesurer les déplacements de l'utilisateur ;
dans lequel l'unité de traitement (6) est en outre configurée pour traiter les déplacements mesurés et les mesures de l'au moins une caractéristique physiologique pour déterminer le risque que l'utilisateur quitte la zone sûre prédéfinie.

12. Appareil (2) selon la revendication 10 ou 11, l'appareil comprenant en outre :
des moyens (14) pour déterminer l'emplacement de l'utilisateur ;
dans lequel l'unité de traitement (6) est en outre configuré pour utiliser l'emplacement déterminé et les mesures de l'au moins une caractéristique physiologique pour déterminer le risque que l'utilisateur quitte la zone sûre prédéfinie.

13. Appareil (2) selon la revendication 10, 11 ou 12, l'appareil comprenant en outre :
un module de mémoire (12) pour stocker des informations relatives à l'heure ou aux heures où un utilisateur est supposé présenter un risque plus élevé de quitter et/ou a déjà quitté la zone sûre prédéfinie ;
dans lequel l'unité de traitement (6) est en outre configurée pour déterminer le risque que l'utilisateur quitte la zone sûre prédéfinie en utilisant les mesures de l'au moins une caractéristique physiologique, l'heure et les informations stockées.

14. Appareil (2) selon l'une quelconque des revendications 10 à 13, dans lequel le capteur (4) pour mesurer au moins une caractéristique physiologique de l'utilisateur est un capteur ou des capteurs pour mesurer au moins l'un parmi la conductance cutanée et le rythme cardiaque de l'utilisateur.

15. Appareil (2) selon l'une quelconque des revendications 10 à 14, l'appareil comprenant en outre :
des moyens (14) pour déterminer l'emplacement de l'utilisateur et un ensemble de circuits émetteurs-récepteurs (8) pour transmettre l'emplacement déterminé au personnel soignant si l'unité de traitement (6) détermine que l'utilisateur présente un risque élevé de quitter la zone sûre prédéfinie.
